# EUROPEAN PATENT APPLICATION

(11) **EP 4 082 600 A1**
(43) Date of publication of application: **02.11.2022**
(21) Application number: 21171638.6
(22) Date of filing: 30.04.2021
(51) Int. Cl.: A61M 39/20, A61M 39/26, A61M 39/10, A61J 1/14, B65D 41/20, A61M 39/00

(54) **VALVE, METHOD OF MANUFACTURING A VALVE, CAP FOR A FLUID CONTAINER COMPRISING SUCH VALVE, FLUID CONTAINER CONTAINING SUCH CAP, AND METHOD FOR MANUFACTURING SUCH CAP**

(71) Applicant: B. Braun Melsungen AG, 34212 Melsungen (DE)
(72) Inventor: VALLOTTON, Raphaël, 1807 Blonay (CH); MILAN, Marco, 1024 Ecublens (CH)
(74) Representative: Prüfer & Partner mbB Patentanwälte · Rechtsanwälte

(57) **Abstract**

Valve (5), comprising a valve housing (51) and an elastic valve member (52) accommodated in the valve housing (51) and configured to close the valve (5) when the valve (5) is in a closed operating state. The valve member (52) comprises a valve opening (522). The valve opening (522) is configured to be closed when the valve (5) is in the closed operating state and to provide fluid passage through the valve (5) when the valve (5) is in an open operating state. The valve member (52) is overmoulded into the valve housing (51). The valve is beneficially used as a valve element of a port of a cap for a fluid container, in particular for a container for a medical fluid.

## Description

The present invention relates to a valve, method of manufacturing a valve, cap for a fluid container comprising such valve, fluid container comprising such cap, and method for manufacturing such cap.

A valve usually has the function to control the flow of fluid through a duct, conduit, or the like. Valves are used in a variety of devices, including a wide range of medical equipment. Herein, valves play a major role which are placed at a connection point of a medical apparatus or a container. Such a connection point, which is also commonly referred to as a "port", is used to connect a fluid transfer device so that a fluid connection can be established between the apparatus or container, respectively, and the fluid transfer device. Such valves can inter alia have the purpose of closing the port when no fluid transfer device is connected thereto, so that no fluid can enter or exit in an uncontrolled manner. Such valves may also serve, among other functions, to establish the fluid connection between the apparatus or container, respectively, and the fluid transfer device and to hold the fluid transfer device in position when connected to the apparatus or container. Valves are used in many different devices in the field of medicine and in other fields. The valve according to the invention is therefore not limited to a valve of a medical apparatus or container. In the field of medical applications, the valve according to the invention may be used, for example, for a wide variety of containers, intravenous sets for infusion or transfusion, transfer devices, etc. Many other uses are possible in the field of medicine and in other fields. Applications of the valve according to the invention may be found, for example, in the field of laboratory equipment and in other fields of science and technology. The valve according to the invention may also be used in devices for personal use.
Valves are used, for example, in the equipment for carrying out infusions and transfusions. Moreover, valves may be installed in ports of containers in which liquids to be used in infusions and transfusions are provided.

For therapeutic purposes, infusions and transfusions are used in human and veterinary medicine. For example, intravenous infusions are used to administer liquids (e.g. solutions of active substances or other liquid pharmaceuticals) into the bloodstream of a patient. For this purpose, the fluid to be administered is taken from a fluid container and flows through an infusion tubing to the intravenous access. The intravenous access can for instance be provided by a peripheral cannula inserted into the patient's median cubital vein. Through the intravenous access, the fluid enters the patient's bloodstream.

An intravenous administration set (also known as "IV administration set", "I.V. administration set", "intravenous set", "infusion set", etc.) includes a flexible tube through which the fluid can flow from the container to the intravenous access. An intravenous administration set is a typical example of an administration device, i.e. a device used for administering a therapeutic fluid to a patient. Often, but not necessarily, the intravenous administration set includes a drip chamber connected to the tube so that the fluid flows from the container through the drip chamber into the tube. The intravenous administration set may optionally include further components, for example a flow regulator such as a roller clamp to control the flowrate of the liquid.

If a drip chamber is present, the drip chamber is connected to the container via a container connection so that the liquid can flow from the container into the drip chamber. Otherwise, the tube is directly provided with a container connector.

The container connector can be a piercing device such as a hollow mandrel which can pierce a septum of the container and which has one or more fluid channels in its interior. Such a piercing device is generally referred to as a "piercing spike" or "spike".

A "septum" is a closure of a container or device in the form of a membrane or comprising a membrane, wherein the membrane is a rubber membrane or a membrane made of another suitable elastic material. Often, the membrane has the form of a disk inserted into the opening of a housing. The membrane is pierced with a hollow piercing device in order to remove fluid from the container or to introduce a fluid into the interior of the container. The piercing device may be a spike as described above, an injection needle, etc. Preferably, the membrane has self-sealing properties. That is when the piercing device is pulled out of the membrane, the opening pierced by the piercing device, i.e. the puncture hole, closes at least partially, preferably completely. The membrane of a septum therefore has no pre-fabricated opening in the form of a hole or slit into which the piercing device is inserted.

A "fluid" is understood to be a flowable material, in particular a liquid or a suspension.

The fluid access of a container or other device is generally referred to as "port" in the context of medical fluid technology. A port is therefore a connection site to which an external device for fluid extraction or fluid addition, hereinafter referred to as "fluid manipulation device", may be connected in order to establish a fluid connection between the interior of the container or the device and the fluid manipulation device. The port of a container for application in infusion or transfusion therapy to which the intravenous administration set can be connected is called "administration port".

Medical fluid containers are containers which are intended to contain medical fluids and are made of a material suitable for this purpose. Medical fluids include liquid pharmaceuticals for therapy or diagnosis, liquid pharmaceutical components, nutrients, blood for transfusion, etc.

The fluid container from which the fluid to be administered is taken may be a bottle made of a rigid material such as glass or rigid plastic. Besides, plastic bottles with a certain degree of flexibility or plastic bags have become established on the market. Plastic not only has the advantage of having a lower specific weight and being less fragile than glass. If a sufficiently flexible plastic is used, a further advantage is that the container collapses when liquid is removed. This means that the volume of the container continuously adapts to the decreasing volume of the liquid in its interior so that no additional pressure equalization measures in the form of an air inlet channel or an air inlet opening are required. This not only simplifies the design of the administration device but also avoids the risk of contamination of the interior of the container by incoming air.

Some of the containers available on the market containing fluids for intravenous administration allow the addition of other drugs (so-called "drug admixture"). For example, the admixture is carried out by injecting the drug via a further port (so-called "medication port") on the container which also includes a septum. That is, the drug is injected by means of an injection syringe fitted with a hollow needle, wherein the needle is pierced through the septum. Usually, the medication port is located at a position on the container close to the position of the administration port. This may make it easier to manufacture the container but more difficult or even impossible to connect a syringe or other fluid manipulation device to the medication port and, at the same time, a piercing spike or other piercing device to the administration device.

Containers for infusion liquids made of a polyolefin material are available on the market. These containers have a cap on the container head. During infusion, the container points downwards. The cap may have a single septum that may have identified piercing points as described, e.g. in the international standard ISO 15759.The cap may have two ports next to each other. A cap of this type is also called a "twin port cap". The two ports may be of the same design. It is also possible that the cap has two septa that are different from each other, either with respect to their material or their design, as shown, e.g. in US 2009/0054865 A1. Each port comprises a septum which may be pierced by the spike of an intravenous administration set or a needle of an injection syringe. The user may deliberately choose which one of the two parts is used as the administration port and which one as the medication port. It is also possible that one of two functionally identical or functionally equivalent ports is intended as the administration port, whereas the other port is intended as medication port, wherein the different intended use may be indicated by using labels, symbols, different colours, etc. For practical reasons, the caps used for medical containers have a limited diameter, which makes the simultaneous connection of medication and administration device difficult or impossible in the case of the known caps.

The injection of a liquid into the container by means of an injection syringe may be cumbersome because a suitable injection needle has to be kept in stock, unpacked, connected to the injection syringe before use and discarded after use. Further, piercing the needle is prone to incorrect handling. In particular if the piercing direction is not vertical to the septum, the injection needle may be bent and thereby damaged and/or the septum may not seal completely after the injection needle has been withdrawn. In addition, handling an injection needle involves a potential risk of injury. If the drug admixture is carried out during the infusion, when the container is then placed upside-down, the port for injecting the drug to be admixtured may be difficult to access. Moreover, the needle is held only in the area of the puncture through contact with the septum material so that the needle and the syringe can be pulled out or fall out unintentionally.

From DE 10 2007 005 407 A1, a cap for a container for medical liquids is known which has an admixture portion and a withdrawal portion. The withdrawal portion has a pierceable membrane through which a spike of an intravenous administration set can be pierced in the conventional manner. The admixture portion is designed to receive the male cone of a syringe. The admixture portion does not have a septum but a slit valve, i.e. a membrane which has a slit that is opened by the inserted male syringe cone. The slit valve is provided by a membrane inserted in the withdrawal portion. The production of such an admixture portion or admixture port is time-consuming and expensive because it involves the production of the body of the cap with the valve seat by means of a first machine, the production of the membrane by means of another machine, and the insertion of the membrane into the valve seat by a further machine. Further, the insertion of the membrane involves the risk of damaging the membrane because of the deformation of the membrane that is necessary therefor. Moreover, when inserting the membrane, particles thereof may be broken or crumbled off. Such particles have to be cleaned off in an additional step because they could cause contaminations otherwise. In addition, there is a gap between the membrane and the wall of the recess because the wall of the recess and the membrane only abut each other. The presence of a gap may not only cause a possible leakage, but is also difficult to disinfect and may even lead to the entry of further impurities during use. This is particularly the case if sections of the slit valve are deformed and folded back or rolled into itself when the cone of a syringe is inserted into the slit of the valve. This can also cause the gap to expand so that, for example, a liquid can easily enter into it. Hence, germs, endotoxins, disinfectants, etc. may accumulate and increase the risk of contaminating the liquid prior to its administration to a patient which can have serious health consequences. Furthermore, as mentioned, the membrane is inserted into a recess, i.e. there only is a form-fit connection. However, since the membrane must be an elastic component in order to fulfil its function, there is a risk that the membrane could be inadvertently pushed out of the valve seat when a syringe cone is inserted. This would render the valve unusable. The cap of DE 10 2007 005 407 A1 and especially the admixture portion (slit valve) is therefore difficult to clean and disinfect. Moreover, the admixture portion and the withdrawal portion are close to each other and point into the same direction so that simultaneous drug admixture and intravenous administration of the liquid are difficult or impossible. For example, the user may have to place a syringe with which he wants to supply a liquid via the admixture portion at an oblique angle, for which the membrane of the admixture port is not designed. The aforementioned problems are all the more serious in this case. Moreover, according to DE 10 2007 005 407 A1, the openings of the administration portion and the withdrawal portion are sealed by break-off parts which are complicated to manufacture and cumbersome to handle.

In view of the situation described above, an objective of the present invention is to provide an improved valve, an improved method of manufacturing a valve, an improved cap for a fluid container, an improved fluid container, and an improved method for manufacturing a cap.

This objective is achieved by the valve according to claim 1, the method of manufacturing a cap according to claim 8, the cap according to claim 9, the fluid container according to claim 14, and the method of manufacturing a cap according to claim 15. Refinements of the invention are specified in the dependent claims. Any feature set forth in the claims dependent on any independent claim as well as any feature set forth in the description of exemplary embodiments of the invention below can be understood as a feature suitable for refining the valve, the cap, the fluid container, and the methods. If possible from a technical point of view, the features of all described embodiments may be combined with one another.

The valve according to the invention is a valve comprising a valve housing and an elastic valve member accommodated in the valve housing and configured to close the valve when the valve is in a closed operating state. The valve member comprises a valve opening, wherein the valve opening is configured to be closed when the valve is in the closed operating state and to provide fluid passage through the valve when the valve is in an open operating state. The valve member is overmoulded into the valve housing. The valve opening, e.g. a slit through the material of the valve member, may be formed in a separate step, for instance a cutting, using for instance a lubricated blade.

The presence of lubricant in the slit may have the further advantage that self-healing of the material, i.e. an unintentional bonding of the cut surfaces, during sterilisation, transport, and storage is reduced or avoided. The presence of lubricant in the slit may have the further advantage that the insertion of a male connector is simplified.

In particular, the valve is in its closed operating state if no fluid manipulation device is connected thereto, and in its open operating state if an appropriate fluid manipulation device such as for example a syringe is connected thereto.

Preferably, the elastic valve member comprises or is made of an elastomeric material.

Due to the overmoulding, the elastic valve member tightly adheres to the inner wall of the valve housing such that there is no gap between the elastic valve member and the valve housing. Such a gap will not open up even if a fluid manipulation device is connected to the valve. Due to the absence of a gap, the risk of leakage is reduced or even avoided. Due to the absence of a gap, also the risk of contamination is reduced or even avoided. In other words, the valve according to the invention is easier to clean and disinfect and less prone to the accumulation of contaminants than conventional valves with a valve opening in an elastic valve member. Hence, the danger emanating from germs, endotoxins, disinfectants, etc. contaminating an infusion liquid or the like prior to its administration is reduced or avoided. Furthermore, due to the tight adhesion, the risk that the valve member could be inadvertently pushed out of the valve housing when a syringe cone or the like is inserted is reduced or avoided.

Due to the absence of a gap between the valve member and the valve housing, bacteria and other contaminant are less likely to accumulate. It may even be possible to manufacture a clean and sterile valve without an additional step of sterilisation using radiation.

Due to the absence of welded part, particle contaminations are less likely to occur or do not occur at all.

In the valve according to the invention, the number of pre-fabricated components is reduced compared to conventional valves. This may reduce the number of assembly steps and manipulations. This may on the one hand improve the efficiency of the manufacturing process. This may on the other hand reduce the risk of component defects and/or contamination.

In addition, it is possible to use a valve member made of a thermoplastic elastomer. Thermoplastic elastomers have excellent material properties and often are recyclable.

Compared to the standard design, the valve according to preferred embodiments of the invention does not comprise portions that are folded back or rolled into itself upon connection of the valve with a male connector. Therefore, only a surface that is easy to clean and disinfect comes into contact with the fluid path. It is therefore easy to efficiently clean and/or disinfect the valve prior to connection with a fluid manipulation device. In the standard design, the whole elastomeric member is compressed downwards into the valve housing, so that a gap will be created. Such gap is difficult to clean and disinfect.

Furthermore, the valve according to the invention is less time-consuming and less expensive to manufacture because forming the valve member and arranging it in the right position are accomplished in one step by overmoulding such that the cumbersome step of inserting a pre-fabricated valve member may be avoided. Hence, also the risk of damaging the valve member because of the deformation used for inserting a pre-fabricated elastic valve member in a valve seat is avoided.

Preferably, the cross-section of the valve member has a T-shape or another shape that ensures that a portion of the slit is by two relatively thin walls, in particular essentially or entirely flat walls. In this case, a pressure applied to the inner side of the valve may for instance push the two walls to each other which may further improve the mechanical fit and tightness.

Preferably, the material of the valve member is selected to ensure (1) easy connection with a male mating connector, (2) sufficient force such that the valve member returns to its initial shape when the male connector is disconnected, (3) resilience, i.e. minimum change of material properties, even after repeated connection of a male connector, (4) high tear resistance to prevent tearing upon insertion of the male connector.

In preferred embodiments the valve member comprises at least one thermoplastic elastomers, preferably at least one polymer selected from the group consisting of styrenic block copolymers, thermoplastic polyolefin-elastomers, thermoplastic vulcanizates, thermoplastic polyurethanes, thermoplastic copolyester, and thermoplastic polyamides.

In addition or alternatively, in further preferred embodiments, the valve member comprises an elastic material having a hardness between 25 and 55 shore A.

In addition or alternatively, in further preferred embodiments, the valve housing comprises a polyolefin, preferably at least one of polypropylene and polyethylene, and/or an engineering plastic, preferably at least one of polyamide, polycarbonate, and polystyrene. Herein, polyolefins are in particular preferred if the valve is used as an element of a cap for a medical liquid container to be used for instance in infusion therapy because polyolefins typically present a cleaner leachable profile, are relatively insensitive to organic solvents that are usually used as disinfectants and are less sensitive to stress-corrosion-cracking than amorphous materials.

In addition or alternatively, in further preferred embodiments, the body comprises a polymer material having a tensile modulus and/or flexural modulus of at least 700 MPa.

Preferably, the valve member is configured to provide a fluid-tight connection with a liquid manipulation device, more precisely with the male connector of the same, so that fluid can flow through the valve exclusively via the connector of the fluid manipulation device. In other words, the connection between the fluid manipulation device and the valve is fluid-tight if it does not leak, i.e. the material around the valve opening is tight against the outer surface of the connector of the fluid manipulation device.

In addition or alternatively, the valve opening preferably is a self-sealing valve opening, i.e. the valve automatically closes after the connection part (male connector, e.g. syringe cone) of the fluid manipulation device has been disconnected. Such self-sealing valve opening may also be referred to as "resealing valve opening".

In particular, the valve opening is formed as a self-sealing channel for a male connector such as a syringe cone. This is preferably achieved by means of a slit formed in the valve member, wherein the slit may be designed in a way to tightly connect to a male connector according to any sub-part of the ISO 80369 series of standards, in particular a male Luer connector according to ISO 80369-7 or a male connector according to ISO 80369-6. The connection system according to ISO 80369-6 is also referred to as NRFit^{®}. Designing a slit according to a connection geometry in compliance with a given standard means that the valve is adjusted to receive a male connector having the dimensions defined in the standard such that a safe fluid connection is established. This ensures compatibility with common medical equipment.

Preferably, the outer surface of the valve member is either essentially flush or entirely flush with an edge surface of the valve housing or projects beyond the valve housing.

The outer surface of the valve member is the surface which points towards the outside of the device or container of which the valve may be an element, i.e. the surface on the side of the valve from which a fluid manipulation device is connected to the valve. The outer surface may also be referred to as the "distal surface" of the valve member.

The expressions "distal" and "proximal" mean closer to or further away from the interior of the device or container of which the valve may be an element. In preferred embodiments, the valve is part of a container cap, wherein the body of the cap has a hat-like structure. The concave side is also referred to as "proximal side" or "inner side" (i.e. the side that points towards the interior of the container if the cap is connected to a container body). The convex side is also referred to as "distal side" or "outer side". In a similar manner, the body's surface on the concave side is also referred to as "proximal surface" or "inner surface" etc.

The edge surface is that portion of the surface of the valve housing which defines the distal opening of the cavity in which the valve member is formed by overmoulding.

The outer surface of the valve member is entirely flush with the edge surface of the valve housing if these two surfaces merge continuously into each other so that, along the entire circumference of the valve member, no step is formed in the area where the surface of the valve member and the surface of the valve housing meet each other. This means that the outer surface of the valve member is in alignment with the edge surface of the valve housing. The outer surface of the valve member is essentially flush with the edge surface of the valve housing if a step of 1 mm or less, preferably 0.5 mm or less, more preferably 0.25 mm, most preferably 0.1 mm or less is formed. In other words: The outer surface of the valve member is entirely flush with the edge surface of the valve housing if neither surface projects or recedes relative to the other surface. The outer surface of the valve member is essentially flush with the edge surface of the valve housing if one of the surfaces in question projects or recedes relative to the other surface, but only by a small offset (1.0 mm or less). It is also possible that the surfaces merge continuously into each other but not along the entire circumference but only along at least 80%, preferably at least 90%, more preferably at least 95% of the circumference of the first seal member. This situation may also be referred to as "essentially flush".

According to a preferred embodiment, the outer surface of the valve member is essentially flush or even entirely flush with the edge surface of the valve housing. Due to such an alignment of the outer surface of the valve member and the edge surface of the valve housing, the process of disinfection of the valve member is made easier and safer. Because no significant discontinuous surface structure such as a step is present in the area to be disinfected, an effective disinfection by applying a disinfectant by rinsing, spraying, wiping, or other application technique can be achieved. The disinfectant can efficiently reach the entire area to be disinfected. This prevents pathogens and other contaminants from accumulating in a surface discontinuity and thus prevents the accumulation of contaminants that cannot be removed effectively or cannot be removed at all by the application of a disinfectant. In addition, a cavity is avoided in which disinfectant could accumulate after application. Such an accumulation of disinfectant ("disinfectant pool") is unfavourable when using the valve because either it is necessary to wait for the evaporation of the disinfectant after disinfection and before connecting a fluid manipulation device with the valve, or there is a risk that disinfectant or other debris (various particulate matter, pathogens, dirt, dead organisms, that cannot evaporate, etc.) is carried through the valve together with the male connector of the fluid manipulation device and could thus enter the patient's bloodstream. Moreover, the disinfection carried out in the course of the production of a cap or a container comprising such a cap may be easier or more efficient due to this alignment of the outer surface of the valve member and the edge surface. In addition, a quick disinfectant evaporation may be beneficial in view of production time and it may avoid that disinfectants are trapped between the valve and a sealing foil applied thereon after disinfection. Disinfectants usually are organic solvents such as ethanol or isopropanol. Such organic solvents could damage the valve in particular if trapped between the valve and the foil. A quicker and more efficient radiation-based disinfection may also be achieved due to an entirely or essentially flush alignment because shadow areas are avoided or minimised.

With regard to disinfection efficiency, it is preferred that the outer surface of the valve member and the edge surface of the valve housing are arranged in an entirely flush manner. However, an only essentially flush alignment, for instance with a small offset of 0.1 to 0.2 mm, is sufficient, or even might be preferred with regard to the robustness of the process of overmoulding. Such small offset, if present, is tolerable with regard to disinfection efficiency.

According to another preferred embodiment, the outer surface of the valve member projects beyond the edge surface of the valve housing, more preferably by 0.1 to 1.0 mm. As described in connection with the flush alignment, convex structures such as recesses around the valve opening are avoided when providing such projection. Hence, a comparable disinfection efficiency is realised.

The method of manufacturing a valve according to the invention comprises the following steps:
A) manufacturing or providing a valve housing,
B) overmoulding an elastic valve member into the valve housing,
D) optionally arranging a peel-off foil atop the valve member.

Preferably, in step A, the valve housing is manufactured through moulding, in particular injection moulding.

According to a preferred embodiment, the method comprises an additional step of cutting a slit in the valve member, preferably by using a lubricated blade.

In particular, a valve according to the invention is manufactured by this method.

Conventional methods of manufacturing valves having an elastic member with a valve opening involve the production of the valve housing by means of a first machine, the production of the elastic valve member by means of another machine, and the insertion of the valve member into a recess of the body by a further machine. This is time-consuming and cost-intensive. Moreover, the insertion of the valve member involves the risk of damaging the valve member because of the deformation that is necessary therefor.

Compared to the conventional methods, the method of the invention may be more time-efficient and more cost-efficient because the valve member is formed in situ by means of overmoulding such that no separate step is required for its insertion. Moreover, the risk of damaging the valve member is reduced by using the method according to the invention. Hence, defect production is reduced and product liability is enhanced by the invention.

The cap according to the invention is a cap for a fluid container. In particular, the cap is a cap for a container for a medical fluid. The cap comprises: (i) a body, (ii) a first port, and (iii) a second port. The body comprises a container connection portion adapted to be tightly connected to a container opening of a container body. The first port comprises a port housing. The first port further comprises an elastic seal member accommodated in the port housing and sealingly closing the port housing. The cap further comprises a second port. The second port comprises a valve according to the invention.

Preferably, the elastic seal member comprises or is made of an elastomeric material.

The cap is adapted to be connected to a container body, i.e. a hollow body with an opening such as for instance a bottle. Whereas a bottle or the like is sometimes referred to as a container, it is referred to as a "container body" here because the expression "container" is used for the capped device in the context of the present invention. Hence, the assembly of the container body and the cap connected thereto is referred to as "container" or "fluid container". For connecting the cap to the container body, the container connection portion of the cap is connected to an opening of the container body referred to as its "mouth". The connection between the cap and the container body may for instance be achieved by fixing the connection portion of the cap to a wall portion of the container body defining the mouth such as a neck portion. For connecting the cap to the container body, various techniques may be used such as, e.g. welding, gluing, and/or screwing. The mouth allows access to the inside of the container body. Apart from the mouth, the container body may comprise further access sites. The shape of the container connection portion of the cap and the portion at the opening of the container body are complementary, so that there is preferably a fluid-tight connection between the container and the cap.

It is also possible that the connection portion of the cap is fixed to an intermediate part connected to the container body, in particular a moulded transition part. For instance, such transition part may be connected on one of its sides to a flexible bag and on its other side to the cap. The cap might have a substantially oval connection portion that is meant to be welded to a baseport that has a matching receiving distal portion on one side and an almond shaped proximal portion on the other side to be welded on the inner side of plastic films of a bag.

Preferably, the elastic seal member of the first port is formed as a pierceable septum. A septum is a closure typically comprising an elastic material such as rubber or other elastomeric material. The septum can be pierced (i.e. penetrated) by a piercing device such as a hollow spike of an infusion set. Such piercing device may also be referred to as "puncture device". The septum seals the first opening, i.e. under normal conditions of use, no fluid can flow through the first opening if the septum has not been pierced by a piercing device. If the septum is pierced by a hollow piercing device, the fluid can flow through a channel within the hollow piercing device, wherein preferably the material of the septum at the piercing site is tight against the outer surface of the piecing device so that no fluid can flow alongside the piercing device but exclusively through the channel of the piercing device. Preferably, the septum is self-sealing, i.e. a channel formed by the piercing device in the material of the septum automatically closes after the piercing device has been withdrawn from the septum, at least if it is pierced by a piercing device up to a certain maximum diameter. This self-sealing function can be achieved for instance by selecting a suitable elastic material. The self-sealing property may also be referred to as "resealing" property.

In particular, the first port is an administration port, wherein preferably the elastic seal member is adapted to be pierced by a spike of an administration device selected from the group consisting of infusion set, transfusion set, and transfer device such that a fluid connection between the interior of a fluid container and the administration device is provided, wherein more preferably the infusion set is an infusion set according to ISO 8536-4, the transfusion set is a transfusion set according to ISO 1135-4, and the transfer device is a transfer device according to ISO 22413. This ensures that the cap is particularly suitable as the cap of a fluid container for infusion or transfusion therapy using conventional equipment such as a conventional infusion set.

The second port comprising the valve according to the invention is a needle-free port, preferably a medication port adapted to provide a fluid connection between the interior of a fluid container and a fluid manipulation device such as a syringe having a male connector, more preferably a male connector according to any sub-part of the ISO 80369 series of standards, most preferably a male Luer connector according to ISO 80369-7 or a male connector according to ISO 80369-6. This ensures that the cap is particularly suitable as the cap of a fluid container for infusion or transfusion therapy with the possibility of drug admixture using conventional fluid manipulation devices such as a conventional syringe without a needle.

Alternatively or in addition, the valve housing is formed monolithically with the body. This simplifies production and ensures that the valve housing is securely anchored so that it cannot unintentionally detach from the remaining body.

Alternatively or in addition, the administration port housing is formed monolithically with the body. This simplifies production and ensures that the port housing is securely anchored so that it cannot unintentionally detach from the remaining body.

According to preferred embodiments of the cap, also the elastic seal member of the first port is formed by overmoulding. This allows the same advantages to be realised as through the overmoulding of the valve member, see above. If not formed by overmoulding, the elastic seal member may for instance be inserted into the first port housing and, preferably, fixed in place. For instance, laser welding can be used to fix the elastic seal member in place.

If also the elastic seal member of the first port is formed by overmoulding, it is possible to form the valve member and the elastic seal member as a single elastic member, i.e. as a single volume of elastic material formed by overmoulding. This allows for a particularly simple manufacture of the cap because both the valve member and the elastic seal member may be formed in one step.

For forming the valve member and the elastic seal member as a single elastic member, it is for instance possible to form a volume of elastic material by overmoulding wherein a portion of the volume serves as valve member and another portion of the volume serves as seal member. It is also possible that the valve member and the seal member are formed as a single volume connected by a sprue. The sprue connects the portion forming the valve member and the portion of the elastic seal member with one another. Such sprue allows for the use of a single injection point lowering the construction and operating costs of the mould.

Preferably, the outer diameter of the port housing is 20 mm or less, more preferably 15 mm or less for good overmoulding properties and good usability of the first port.

Preferably, the elastic seal member or the first port is partially covered by a distal portion of the port housing. This helps controlling the deformation of the elastic seal member upon connection and disconnection of a piercing device, i.e. the force required for connection and disconnection as well as the elastic seal member's ability to reseal. The distal surface portion of the elastic seal member that is not covered by a distal portion of the port housing is referred to as "distal surface" of the elastic seal member.

Preferably, the outer surface of the elastic seal member is essentially flush or entirely flush with an edge surface of the port housing.

The outer surface of the elastic seal member (septum) is the surface which points towards the outside when the cap is connected to a container body, i.e. the surface which does not point towards the inside of the container and which also does not abut the housing. The outer surface may also be referred to as the "distal surface" of the elastic seal member. Specifically for a container cap, the expressions "distal" and "proximal" mean closer to or further away from the interior of the container body in an assembled state in which the cap is connected to the container body.

The edge surface of the port housing is that portion of the surface of the port housing which defines the distal opening of the cavity in which the elastic seal member is accommodated, in particular formed by overmoulding. Therefore, the edge surface defines the border of the opening. In preferred embodiments, the port housing is formed as a hollow protrusion protruding from the remaining body of the cap. The elastic seal member is accommodated in the hollow protrusion. The edge surface of the port housing, viewed in a distal-proximal direction, is the front surface of the protrusion.

The outer surface of the elastic seal member is entirely flush with the edge surface of the port housing if these two surfaces merge continuously into each other so that, along the entire circumference of the elastic seal member, no step is formed in the area where the surface of the elastic seal member and the surface of the port housing meet each other. This means that the outer surface of the elastic seal member is in alignment with the edge surface of the port housing. The outer surface of the elastic seal member is essentially flush with the edge surface of the port housing if a step of 1 mm or less, preferably 0.5 mm or less, more preferably 0.25 mm, most preferably 0.1 mm or less is formed. In other words: The outer surface of the elastic seal member is entirely flush with the edge surface of the port housing if neither surface projects or recedes relative to the other surface. The outer surface of the elastic seal member is essentially flush with the edge surface of the port housing if one of the surfaces in question projects or recedes relative to the other surface, but only by a small offset (1 mm or less).

Due to such an alignment of the outer surface of the elastic seal member and the edge surface of the port housing, the process of disinfection of the elastic seal member is made easier and safer. Hence, this allows the same advantages to be realised as through a flush alignment of the outer surface of the valve member and the edge surface of the valve housing, see above.

With regard to disinfection efficiency, it is preferred that the outer surface of the elastic seal member and the edge surface of the port housing are arranged in an entirely flush manner. However, an only essentially flush alignment, for instance with a small offset of 0.1 to 0.2 mm, is sufficient, or even might be preferred with regard to the robustness of the process of overmoulding. Such small offset, if present, is tolerable with regard to disinfection efficiency.

Due to the provision of both ports on the same base part (body) of the cap, an efficient fabrication of fluid containers is possible because the container body has to be connected to only one part. In addition, the body may be adapted to different container mouth shapes. This is in particular the case because according to the invention, an efficient fabrication of the cap is possible.

The valve of the cap according to the invention provides a connection between the interior of a container body connected to the cap and a fluid manipulation device. In this case, a fluid manipulation device is a device with which fluid such as a liquid can be removed from the container or with which fluid can be added to the container. The fluid manipulation device may be, for example, a syringe without a needle attached. The valve opening is configured to be connected to the male connector part of the fluid manipulation device. Fluid transfer is then possible between the interior of the container body connected to the cap and the fluid manipulation device. Preferably, the connection between the male connector part of the fluid manipulation device and the valve opening is fluid-tight, i.e. fluid can flow through the valve exclusively via the male connector of the fluid manipulation device.

Preferably, the valve opening is resealable, i.e. after disconnecting the fluid manipulation device, the valve opening automatically closes such that the valve member seals the valve opening in a fluid-tight manner again.

In preferred embodiments of the cap according to the invention, the first port is an administration port, i.e. a port for withdrawing a liquid that is to be administered to a patient from the container body connected to the cap. For instance, the elastic seal member is adapted to be pierced by a spike of an administration device such as an infusion set, a transfusion set, a transfer device etc. By piercing the elastic seal member by the spike, a fluid connection between the interior of a container body connected to the cap and the administration device is established.

In addition or alternatively, in further preferred embodiments of the cap, the second port is a needle-free port, i.e. a port through which a fluid connection with a syringe that is not provided with a needle or a similar device can be made. In other words, the second port is not intended to be pierced by a needle or another device in order to provide a fluid connection via the second port. It is preferred that the fluid connection between the interior of the container body connected to the cap and the syringe or the other fluid manipulation device is a bidirectional fluid connection, i.e. fluid can flow through the valve opening either into the container body or out of the container body.

In addition or alternatively, in further preferred embodiments, the outer surface (distal surface) of the elastic seal member is essentially flat or entirely flat. Herein, the outer surface is considered to be essentially flat if it has protrusions and/or recesses whose height is less than 10% of the largest diameter of the outer surface. For instance, it is possible that the outer surface has such a recess in order to indicate the location where the user should pierce the elastic seal member.

Preferably, the diameter of the elastic seal member (measured transversely to the direction in which it is pierced) is large enough such that the elastic material in the space between a piercing device such as a spike pierced through the elastic seal member and the port housing does not need to be squeezed upon insertion of the piercing device thus preventing high friction to the piercing device being inserted and allowing for easy insertion of the piercing device.

Preferably, the diameter of the elastic seal member (measured transversely to the direction in which it is pierced) is small enough such that a sufficient force for holding the piercing device is ensured.

In addition or alternatively, in further preferred embodiments, the valve member has a diameter measured transversely to the direction in which it is pierced of at least 8 mm, preferably at least 9 mm and/or 13 mm or less, preferably 11 mm or less.

The elastic seal member may also have a non-circular shape. It may, e.g., be elliptical. The above-mentioned preferred diameters are then understood as the smaller dimension of the non-circular shape, e.g. the minor axis of an ellipse.

Preferably, the thickness of the elastic seal member (measured in the direction in which it is pierced) is chosen to ensure sufficient resealability and sufficient force for holding the piercing device, and to avoid excessive force for the insertion of the piercing device.

In addition or alternatively, in further preferred embodiments, the thickness of the elastic seal member measured in the direction in which it is pierced is 1.5 mm or more, preferably 2 mm or more and/or 5 mm or less, preferably 4 mm or less.

Preferably, the material of the elastic seal member is chosen to ensure sufficient resealability and sufficient force for holding the piercing device, and to avoid excessive force for the insertion of the piercing device.

Preferably, the valve member has a cross-section having a T-shape. Such valve member does not fill the entire cavity within the valve housing. Instead, the only a distal valve portion extends over the cross-sectional area in the valve housing and thus closes off the valve housing on this side, so that fluid can only pass through the valve opening - provided that the valve is in the open operating state. That is, the distal portion, having for instance a disk-like shape, fills the housing inner diameter and is circumferentially bonded to the valve housing without interruption. In this way, leakage around the valve member is prevented and resistance towards shear upon connection is improved. A proximal portion of the valve member is connected to the distal portion, wherein the proximal portion may have the form of a ridge extending in the direction of the diameter of the valve housing. In a section in a plane perpendicular to the ridge, the T-shape of the valve member is visible. Such T-shapes may make it, inter alia, possible to provide a long slit which therefore has excellent resealing properties and to save material at the same time.

In addition or alternatively, in further preferred embodiments, the elastic seal member comprises at least one thermoplastic elastomer, more preferably at least one thermoplastic elastomer selected from the group consisting of styrenic block copolymers, thermoplastic polyolefin elastomers, thermoplastic vulcanizates, thermoplastic polyurethanes, thermoplastic copolyester, and thermoplastic polyamides.

In addition or alternatively, in further preferred embodiments, the elastic seal member comprises an elastic material having a hardness between 25 and 55 shore A.

In addition or alternatively, the elastic seal member comprises an elastic material including pigments and/or dyes capable of absorbing electromagnetic radiation. The presence of substances having the ability of absorbing electromagnetic radiation may for instance improve the usability of laser welding for connecting the elastic seal member to the port housing. It is, however, preferred that also the elastic seal member is formed by overmoulding such that no separate step is required for connecting the elastic seal member to the port housing.

In addition or alternatively, in further preferred embodiments, the diameter of the valve member (measured transversely to the direction in which the fluid manipulation device is connected to the second port) is 13 mm or less, preferably 11 mm or less, and/or 8 mm or more, preferably 9 mm or more. In those embodiments of the invention in which the valve housing comprises an external thread structure, the diameter of the valve housing and in turn also the diameter of the valve member is limited by the diameter of the mating internal thread of the fluid manipulation device with which a connection is intended.

The valve member may also have a non-circular shape. It may, e.g., be elliptical. The above-mentioned preferred diameters are then understood as the smaller dimension of the non-circular shape, e.g. the shorter axis of an ellipse.

In addition or alternatively, in further preferred embodiments, the length of the valve member (measured in the direction in which the fluid manipulation device is connected to the second port) is between 3 and 12 mm. preferably between 5 and 10 mm. Herein, the length may be selected such that it is at least a minimum length ensuring tightness after disconnection of the fluid manipulation device. The maximum length may depend on the length of the male connector. For the Luer geometry and similar designs of the male connector, a length between 3 and 12 mm will usually be appropriate. For other connector designs, a different length may be more appropriate.

In addition or alternatively, in further preferred embodiments, the body comprises a polyolefin, preferably at least one of polypropylene and polyethylene, and/or an engineering plastic, preferably at least one of polyamide, polycarbonate, and polystyrene.

In addition or alternatively, in further preferred embodiments, the body comprises a polymer material having a tensile modulus and/or flexural modulus of at least 700 MPa.

In addition or alternatively, in further preferred embodiments, the valve member provides a fluid-tight connection with the fluid manipulation device, in particular a releasable connection such that the fluid-tight connection and disconnection of a fluid manipulation device is possible multiple times.

The valve housing of the second port may comprise a thread structure, preferably a thread structure adapted for connecting with a male Luer lock structure.

Preferably the portion of the valve housing that is adapted with the thread structure has a length (measured in the axial, i.e. distal-proximal direction) of at least 3.5 mm, such that the thread structure is long enough to ensure safe connection of the lock structure and short enough for a convenient number of revolutions for closing the lock connection in the sense of increased usability. The length of the thread portion may be selected according to the design of the fluid manipulation device with which a connection is intended. For the Luer lock geometry and similar designs of the male connector, a length between 3.5 mm and 5.5 mm will usually be appropriate. For other connector designs, a different length may be more appropriate.

Preferably, the distal portion of the valve housing, i.e. the portion that may be provided with a thread structure, has a diameter of 18 mm or less, more preferably 13.5 mm or less.

Preferably, the cap has a height between 12 mm and 50 mm, more preferably between 14 mm and 30 mm, measured in a direction transverse to the mouth of the container body 200 with which the cap 1 is to be connected. Such height may be seen as a compromise between a user-friendly handling and a low residual volume within the cap 1 and space requirements during transport and storage.

Preferably, the proximal-distal direction for the first port and the proximal-distal direction for the second port are not parallel to each other but enclose an acute angle, more preferably an angle between 15 and 45 degrees, even more preferably between 25 and 35 degrees. In other words, the first port and the second port are preferably in an angled arrangement. The angled arrangement of the first port and the second port makes it possible or at least easier that a piercing device and a fluid manipulation device are simultaneously connected to the first port and second port, respectively. Forming the valve member and optionally also the elastic seal member by the overmoulding technique is advantageous if an angled arrangement of the ports is intended because the insertion of pre-fabricated elastic members in different directions would be particularly cumbersome.

When a container being provided with the cap according to the invention is used for administering a liquid to a patient, the container and the cap are usually oriented such that the cap is at the bottom. Hence, the proximal-distal direction for the first port is vertical whereas the proximal-distal direction for the second port is inclined. The inclined orientation of the latter may make it easier for the user to administer a substance through the second port, e.g. by using a syringe.

Optionally, a peel-off foil is arranged atop the elastic seal member and/or atop the valve member of the cap, in particular by welding the peel-off foil to the port housing or the valve housing, respectively.

The peel-off foil is removed before a piercing device is pierced through the elastic seal member or before a male connector is inserted into the valve opening, respectively. The peel-off foil seals and protects the respective port, and in particular provides tamper evidence because the peel-off foil makes it easy to determine whether the respective elastic member is damaged or has already been connected to a piercing device or male connector, respectively. Furthermore, the peel-off foil provides additional tightness for the respective port and helps to ensure its mechanical integrity, for example, when the cap is subjected to mechanical stress. Compared to a break-off part, a peel-off foil is less subject to damage. This is due to its flat construction. Further, less material is needed for a peel-off foil. Further, the use of a peel-off foil leads to a shorter container, which is advantageous for storage and transportation.

The fluid container according to the invention, which in particular is a fluid container for a medical fluid, comprises
- a hollow container body having a container body opening portion
- a cap according to the invention.

The body of the cap is in fluid-tight connection with the opening portion.

The fluid container may comprise a cap of any one of the embodiments of the invention.

A further method according to the invention is a method of manufacturing a cap for a fluid container, in particular for a container for a medical fluid. The cap to be manufactured comprises a body. It further comprises a first fluid port with a port housing and an elastic seal member. It further comprises a second fluid port comprising a valve with a valve housing and an elastic valve member. The method comprises the following steps:
A) manufacturing or providing the body, the port housing, and the valve housing, preferably manufacturing or providing the body with which at least one of the port housing and the valve housing is integrally formed,
B) overmoulding the elastic seal member into the port housing,
C) overmoulding the valve member into the valve housing,
D) optionally arranging a peel-off foil atop the seal member and/or atop the valve member.

Preferably steps B and C are at least in part simultaneously performed. This may for instance enhance the time-efficiency. Otherwise, step C may be carried out prior to step B or vice versa.

More preferably the elastic seal member and the valve member are formed as a single volume, optionally joined by at least one sprue.

According to a preferred embodiment, the mentioned further method comprises an additional step of cutting a slit in the valve member, preferably by using a lubricated blade.

Other features and expediencies of the invention may be found in the description of exemplary embodiments with the aid of the appended drawings.
- Fig. 1: shows a schematic side view of a valve according to a first embodiment of the invention.
- Fig. 2: shows a schematic side view of the valve according to the first embodiment of the invention in a perspective that is rotated by 90° in relation to the perspective of Fig. 1.
- Fig. 3 (a): shows a schematic top view of the valve according to the first embodiment of the invention.
- Fig. 3 (b): shows a schematic bottom view of the valve according to the first embodiment of the invention.
- Fig. 4 (a): shows a perspective view of the valve according to the first embodiment of the invention from diagonally above.
- Fig. 4 (b): shows a perspective view of the valve according to the first embodiment of the invention from diagonally below.
- Fig. 4 (c): shows a further perspective view of the valve according to the first embodiment of the invention from diagonally below.
- Fig. 5: shows a schematic sectional view of the valve according to the first embodiment as in plane A-A, which is indicated in Fig. 1.
- Fig. 6: shows a schematic sectional view of the valve according to the first embodiment in plane C-C, which is indicated in Fig. 2.
- Fig. 7: shows a schematic side view of a valve according to a second embodiment of the invention.
- Fig. 8: shows a schematic side view of the valve according to the second embodiment of the invention in a perspective that is rotated by 90° in relation to the perspective of Fig. 7.
- Fig. 9 (a): shows a schematic top view of the valve according to the second embodiment of the invention.
- Fig. 9 (b): shows a schematic bottom view of the valve according to the second embodiment of the invention.
- Fig. 10 (a): shows a perspective view of the valve according to the second embodiment of the invention from diagonally above.
- Fig. 10 (b): shows a perspective view of the valve according to the second embodiment of the invention from diagonally below.
- Fig. 10 (c): shows a further perspective view of the valve according to the second embodiment of the invention from diagonally below.
- Fig. 11: shows a schematic sectional view of the valve according to the second embodiment in plane A-A, which is indicated in Fig. 7.
- Fig. 12: shows a schematic sectional view of the valve according to the second embodiment in plane C-C, which is indicated in Fig. 8.
- Fig. 13: shows a schematic sectional view of a cap according to a further embodiment of the invention.
- Fig. 14: shows a schematic perspective view of the cap according to the embodiment shown in Fig. 13 from diagonally below.
- Fig. 15: shows a schematic sectional view of a fluid container according to a further embodiment of the invention, wherein the container comprises a cap according to the embodiment shown in Figures 13 and 14.

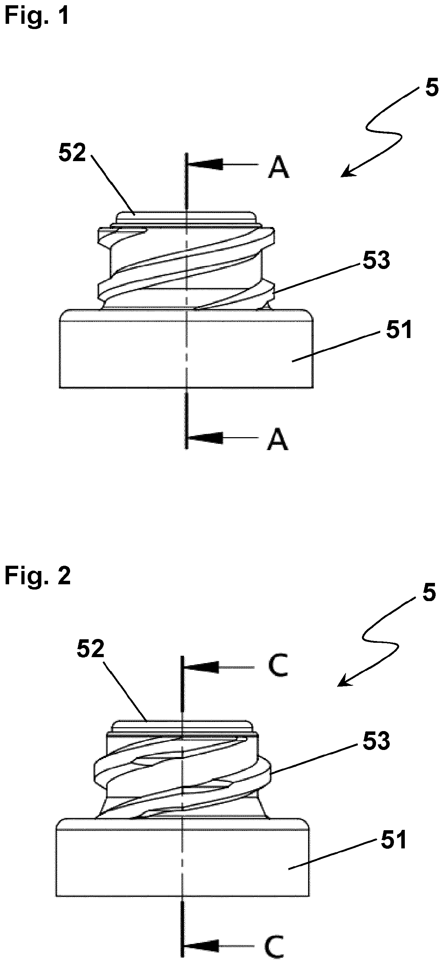

Figures 1 to 6 show a valve 5 according to a first embodiment of the invention in different views. In connection with the description making reference to these figures, expressions such as "top", "bottom", "above", and "below" refer to the orientation shown in Figs. 1, 2, 5, and 6 without loss of generality. The top side is also referred to as the "distal side" and the bottom side as the "proximal side".

The valve 5 comprises a valve housing 51. Preferably, the housing comprises a plastic material such as a polyolefin and/or an engineering plastic, wherein in particular polyethylene and/or polypropylene are used as polyolefin.

In the embodiment shown in Figures 1 to 6, the valve housing 51 has an essentially cylindrical lower portion and upper portion with an essentially cylindrical inner wall. The upper portion has a smaller diameter that the lower portion. The lower portion and the upper portion are connected by a tapered intermediate portion such that the valve housing 51 has closed lateral walls but is open at the top and bottom. This shape is only exemplary. In further embodiments not shown in the figures, the valve housing may for instance have non-circular diameters. Irrespective of its specific shape, it is preferred that the valve housing 51 has closed lateral walls and is at least partially open at the top and at the bottom side such that the valve housing 51 defines a passageway between the open sides.

The bottom of the valve housing 51 may be connected to any device such as an apparatus or container cap so that a fluid transfer into or out of the device is possible through the valve 5.

The valve 5 further comprises a valve member 52. The valve member 52 comprises a valve opening 522. In Figures 1 to 6, the valve 5 is shown in its closed operating state. In this state, the valve opening 522 is closed so that valve member 52 blocks the valve, i.e. fluid is prevented from flowing through the passageway defined by the valve housing 51.

In a further operating state ("open operating state") which is not shown in the figures, the valve opening 522 is open and allows a fluid to flow through the passageway defined by the valve housing 51. Herein, the cross-sectional area of the open valve opening 522 may be smaller than the cross-sectional area of the passageway.

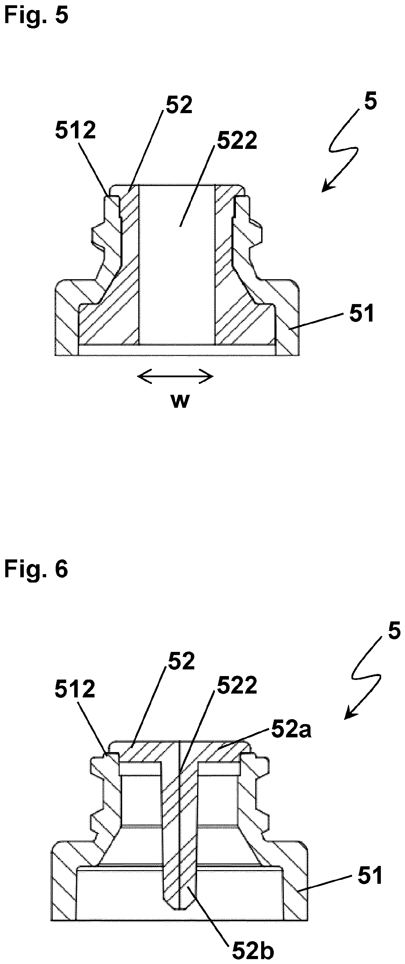

In the first embodiment, the valve member 52 does not fill the entire cavity within the valve housing 51. Instead, the only a distal valve portion 52a extends over the cross-sectional area in the valve housing 51 and thus closes off the valve housing 51 on this side, so that fluid can only pass through the valve opening 522 - provided that the valve 5 is in the open operating state. That is the distal portion 52a, shown as a disk-like portion in Fig. 6, fills the housing inner diameter and is circumferentially bonded to the valve housing without interruption. In this way, leakage around the valve member is prevented and resistance towards shear upon connection is improved. A proximal portion 52b of the valve member 52 is connected to the distal portion 52a, wherein the proximal portion 52b is in the form of a ridge extending in the direction of the diameter of the valve housing 51. In a section in a plane perpendicular to the ridge, the valve member 52 has a T-shape. Such T-shapes may make it, inter alia, possible to provide a long slit which therefore has excellent resealing properties and to save material at the same time.

In further embodiments that are not shown in the figures, the valve member 52 has a different shape. It may for instance fill the entire cavity within the valve housing 51.

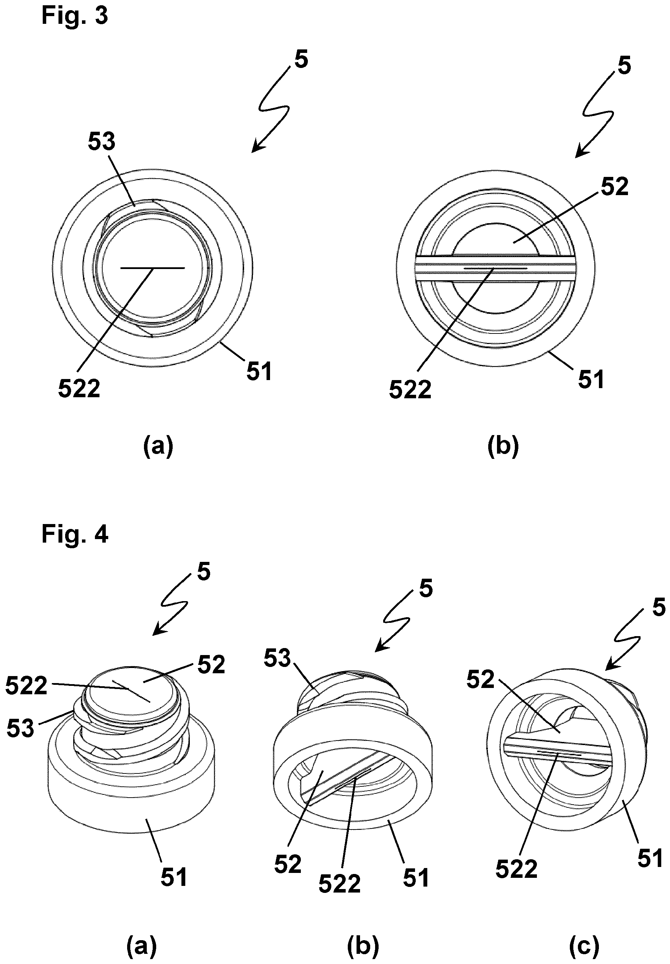

The valve member 52 comprises a valve opening 522. In the case of the first embodiment, the valve opening 522 is a slit formed in the valve member. The slit has the width w (Fig. 5). The slit extends over the entire axial length (vertical direction in Figures 1, 2, 5, and 6) of the valve member. The distal or the proximal end of the slit is visible in Figures 3 (a) to 4 (c). The slit lies in the plane A-A shown in the sectional view of Fig. 5. Accordingly, the slit is perpendicular to the plane C-C shown in the sectional view of Fig. 6.

Preferably, the valve opening 522 is adapted to receive a male connector of a fluid manipulation device such as the cone of a syringe or the like. That is, the male connector can be inserted into the valve opening in an axial direction, i.e. in the direction from the distal side to the proximal side ("distal-proximal direction").

By inserting an appropriate male connector, the valve 5 turns from the closed to the open operating state, i.e. the male member spreads the walls of the slit, which are in contact with each other in the closed operating state.

The valve member 52 is formed by overmoulding, i.e. the material of the valve member 52 is injected into the valve housing 51 by using a moulding technique.

According to the first embodiment, the distal surface 521 of the valve member 52 projects further than the distal end of the valve housing 51, i.e. further than the edge surface 512 of the valve housing. In this way, the upper part of the distal portion 52a forms a projecting cap atop the open distal side of the valve housing 51. As can be seen in particular from Figures 5 and 6, the cover may lap over the distal edge surface 512 of the valve housing 51, for example in order to additionally anchor the valve member 52 in the valve housing 51 and hold it in position. Such overlap over the distal edge surface, however, is optional.

As shown in Figures 1 to 6, the valve housing comprises an external thread structure 53. The thread structure 53 is adapted to engage with a corresponding thread structure located at a male connector.

The thread structure 53 and the corresponding thread structure provide means for locking the connection between the valve and the male connector. The design of the thread structure 53 depends on the design of the corresponding thread structure of a fluid manipulation device or other device with which a connection to the valve 5 is intended. Preferably, the thread structure 53 is designed according to any of the sub-parts of the ISO 80369 series of standards because connection structures according to these standards are frequently used in medical fields.

According to further embodiments of the invention that are not shown in the figures, different structures for locking the connection with a male connector having a corresponding locking structure are provided, such as means for a snap connection. According to further embodiments of the invention that are not shown in the figures, no structures for locking the connection are provided at all so that the male connector is to be held in place exclusively by the force exerted by the valve member which may be sufficient for many applications.

The valve member 52 comprises a valve opening 521, wherein preferably the valve member and the valve opening 521 are configured to provide a fluid-tight connection with a male mating connector of a fluid manipulation device such as a syringe without a needle attached. The valve opening 521 of the embodiment shown in Figures 1 to 6 is formed by a single slit 522 in a cutting plane along the axial direction. In alternative embodiments that are not shown in the figures, several slits may be provided whose cutting planes intersect each other along an axial line. In a section transverse to the axial direction, the cuts then form a cross or a star. Further, for instance a H-shaped section or other shapes of the valve opening may be possible.

The configuration and the size of the valve opening depends on how the connection part, i.e. the male connector, of the device with which a connection is intended is formed. If the connection with a conventional syringe is to be possible, the valve opening may, for example, be formed as a slit 522 as explained above.

Preferably, the valve opening 521 is resealable, i.e. after disconnecting the fluid manipulation device, the valve opening closes 521 such that the elastic valve member 52 ensures that the valve 5 is closed in a fluid-tight manner again. More preferably, this resealing property is retained if a fluid manipulation device is connected and disconnected again several or many times.

In specific examples of the embodiments and its possible modifications described above, the valve member 52 comprises at least one thermoplastic polymer, preferably at least one polymer selected from the group consisting of styrenic block copolymers, thermoplastic polyolefin-elastomers, thermoplastic vulcanizates, thermoplastic polyurethanes, thermoplastic copolyester, and thermoplastic polyamides.

In addition or alternatively, the valve member 52 comprises an elastic material having a hardness between 35 and 45 shore A.

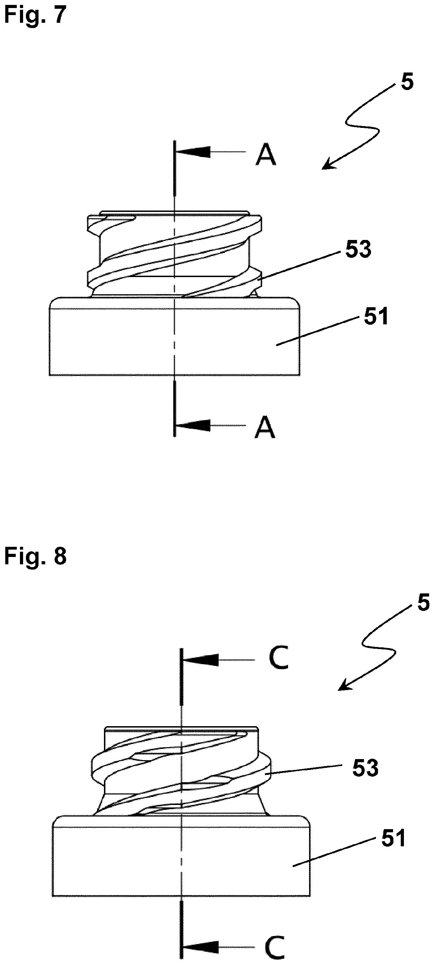

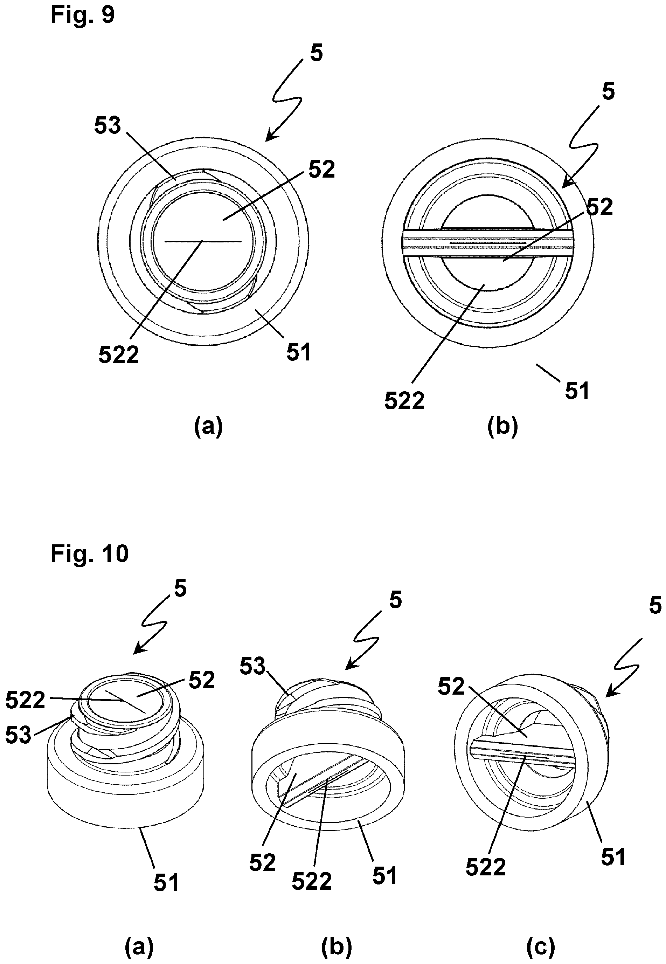

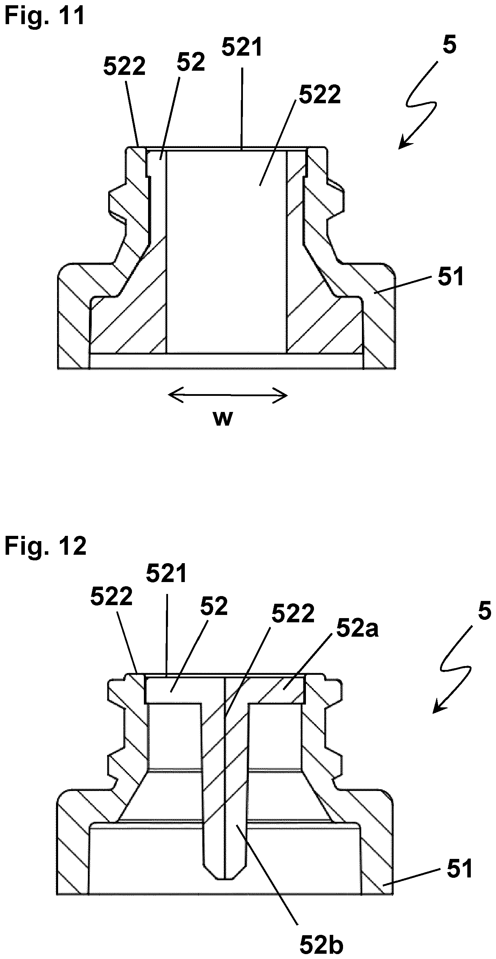

Figures 7 to 12 show a valve 5 according to a second embodiment of the invention in different views. In connection with the description making reference to these figures, expressions such as "top", "bottom", "above", and "below" refer to the orientation shown in Figs. 7, 8, 11, and 12 without loss of generality. The top side is the distal side and the bottom side is the proximal side.

Most of the features of the valve 5 according to second embodiment are equal to the features of the valve 5 according to the first embodiment so that reference is made to the description of the first embodiment above. Hence, the same reference signs are used for both embodiments. Moreover, the modifications discussed for the first embodiment, e.g. with respect to the thread structure 53 or its possible absence, are also possible in the case of the second embodiment.

The second embodiment differs from the first embodiment in that the distal surface 521 of the valve member 52 does not project further than the distal end of the valve housing 51 so that the distal surface 521 is entirely flush or essentially flush with the distal edge surface 512 of the valve housing 51.

The distal surface 521 of the valve member 52 is entirely flush with the edge surface 512 of the valve housing 51 if these two surfaces merge continuously into each other so that, along the entire circumference of the valve member 52, no step is formed in the area where the surface of the valve member 52 and the surface of the valve housing 51 meet each other. This means that the outer surface of the valve member 52 is in alignment with the edge surface 512 of the valve housing 51. The distal surface 521 of the valve member 52 is essentially flush with the edge surface 512 of the valve housing 51, if a step of 1 mm or less, preferably 0.5 mm or less, more preferably 0.25 mm, most preferably 0.1 mm or less is formed between these surfaces. In other words: The distal surface 521 of the valve member 52 is entirely flush with the edge surface 512 of the valve housing 51 if neither surface projects or recedes relative to the other surface. The distal surface 521 of the valve member 52 is essentially flush with the edge surface of the valve housing 51 if one of the surfaces in question projects or recedes relative to the other surface, but only by a small offset (1 mm or less).

Due to such an alignment of the distal surface 521 of the valve member 52 and the edge surface 512 of the valve housing 51, the process of disinfection of the valve member 52 is made easier and safer.

In the specific embodiment shown in Figures 7 to 12, the distal surface 521 of the valve member 52 and the edge surface 512 of the valve housing 51 are essentially flush with one another as there is only a minor step of less than 0.5 mm.

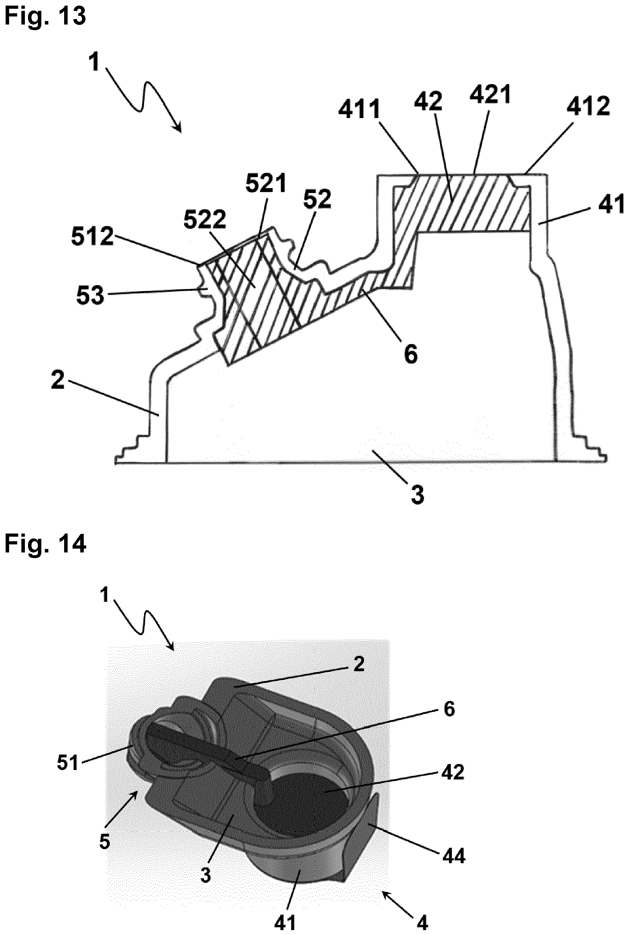

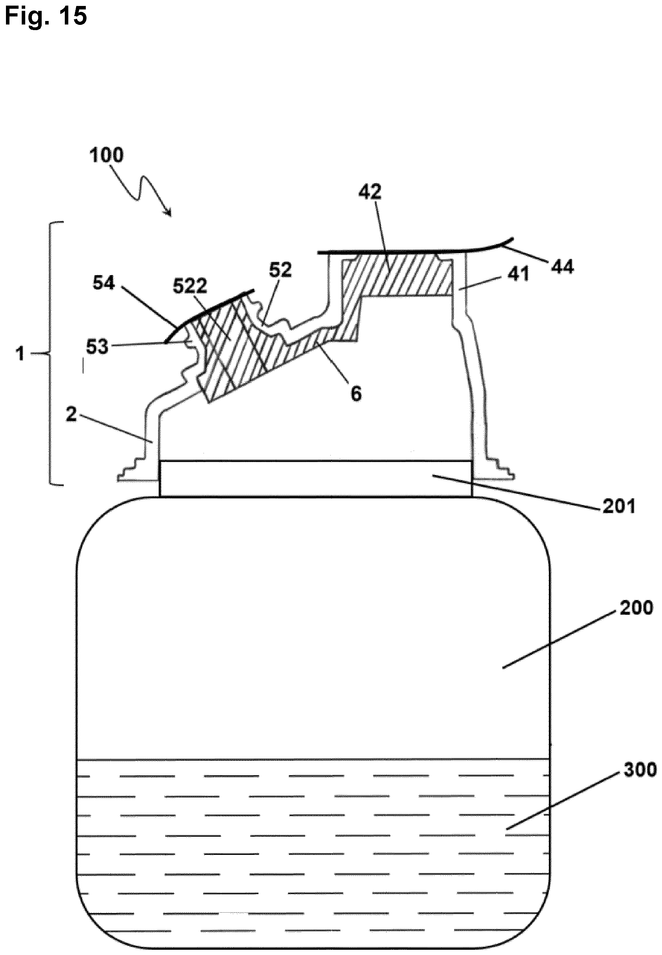

Figures 13 and 14 are a schematic view of a cap 1 according to a further embodiment of the invention. The cap 1 is intended to be attached to the opening 201 of a container body 200. Together, the cap 1 and the container body 200, onto which the cap is mounted as a container closure, form a container 100 according to the invention. The container 100 including a cap 1 according to the embodiment shown in Figures 13 and 14 is shown in Fig. 15. The container 100 shown in Fig. 15 contains a liquid 300 that is not part of the container 100. The opening 201 of the container body 200 is formed as a container neck, of which an axial portion is enclosed by a connection portion 3 of the cap 1. Other configurations of the container body's opening structure and the connection portion are possible, e.g. a neck that is entirely enclosed by the connection portion 3 or a neck-less opening to a front surface of the cap 1 serving as connection portion 3 is attached by glue, welding, etc.

Preferably, the fluid 300 is a medical fluid such as a liquid drug. In other words, it is preferred that the container 100 is suitable to contain a medical fluid and suitable to be used in a medical environment.

Preferably, the container 100 is tightly closed if both ports are in their closed state, i.e. if neither an administration device nor a fluid manipulation device is connected to the respective port. In use, e.g. during administering an intravenous infusion, the container 100 is usually in an upside-down orientation, that is, the cap 1 is then located at the lowermost position so that the liquid 300 can flow from the container body 200 into the administration device connected to the first port 4. For adding a fluid to the inside of the container 100 through the second port 5 using a fluid manipulation device, in general no specific orientation of the container 100 is required.

The cap 1 comprises a body 2 constituting the main part of the cap 1. In the illustrated embodiment, the body 2 is shown as a hollow cup-like structure with
- an opening at the connection portion 3 on the bottom side in the orientation shown and
- protrusions positioned on an elevated section on the body's upper side in the portions where the ports 4, 5 (see below) are formed.

The elevated section and the protrusions are optional.

In the illustrated embodiment, the lower part of the cap 1 is circular because the cap 1 according to this embodiment is intended to be attached to a container body 200 with a circular opening 201. Other shapes of the lower part of the cap 1 are possible for other shapes of the opening, e.g. elliptical or rectangular with rounded corners.

In the illustrated embodiment, the connection portion 3 is shown to be a tapered ring-like portion of the inner surface of the cap 1. In other embodiments, the connection portion may include, for instance, a cylindrical inner surface portion, an inner surface portion with a step, a front surface at the lower edge of the cap 1, etc. It is also possible that the connection portion 3 is deformed when the cap 1 is connected to the container body 200, in particular if welding or another heat-involving process is used therefor.

The cap comprises two ports 4, 5. The first port 4 may, e.g., serve as administration port. The second port 5 may, e.g., serve as medication port.

Both ports 4, 5 provide access points for fluid transfer into or out of the container 100 if the cap 1 is connected to a container body 200.

The piercing device and the fluid manipulation device are not part of the container according to the present invention.

The second port 5 comprises or is constituted by a valve 5 according to the invention.

Fig. 13 is a sectional view wherein the sectional plane coincides with the plane in which the valve opening 522 formed as a slit lies, so that the slit is drawn as a hatched rectangular area within the valve member 52.

Preferably, the valve housing 51 is formed monolithically with the body 2 of the cap 1 because in this way, both the body 2 and the valve housing 51 may be formed in a single manufacturing step, for instance by using an injection moulding technique. Alternatively, the valve housing 51 is not formed monolithically; instead, at least a portion of the valve housing is formed as an element that is attached to the remaining body of the cap, for instance by laser welding. With respect to the preferred features of the valve 5 being part of the cap 1, reference is made to the above description of preferred features and embodiments of the valve.

The first port 4 comprises an elastic seal member 42 accommodated in the port housing 41 and sealingly closing the port housing 41.

In the embodiment shown in Figures 13 and 14, the port housing 41 is a hollow circular cylindrical protrusion formed monolithically with the body 2, wherein the upper edge of the port housing 41, i.e. the circular front surface 412, defines an opening 411.

In further embodiments, the cylindrical protrusion has a non-circular cross section, e.g. an elliptical cross section. In further embodiments, the protrusion is conical.

In further embodiments, the port housing is not formed monolithically with the body; instead, at least a portion of the port housing is formed as a element that is attached to the remaining body of the cap, for instance by laser welding.

In the port housing 41, an elastic seal member 42 is accommodated. According to this embodiment, the elastic seal member 42 is formed as a pierceable septum 42 that may be pierced by a spike of an infusion set or another piercing device. That is, the septum 42 closes the opening of the first port 4 if no piercing device has been pierced through the septum 42. If a hollow piercing device is pierced through the septum 42, a fluid can flow through the piercing device. In other words, the piercing device pierced through the septum 42 acts as fluid entrance or fluid exit of a container 100 if the cap 1 is connected to a container body 200.

Preferably, the septum 42 is self-sealing, i.e. a channel formed by the piercing device in the material of the septum 42 automatically closes after the piercing device has been withdrawn from the septum 42.

Preferably, the size of the septum 42 measured transversely to the direction in which it is pierced is large enough such that the elastic material in the space between a piercing device such as a spike pierced through the septum 42 and the port housing 41 does not need to be squeezed to much upon insertion of the piercing device thus preventing high friction to the piercing device being inserted and allowing for easy insertion of the piercing device. Further preferably, the size of the septum 42 measured transversely to the direction in which it is pierced is small enough such that a sufficient force for holding the piercing device is ensured.

In addition or alternatively, in further preferred embodiments, the septum has a diameter measured transversely to the direction in which it is pierced of at least 8 mm, preferably at least 9 mm and/or 13 mm or less, preferably 11 mm or less.

Preferably, the thickness of the septum 42 measured in the direction in which it is pierced is chosen to ensure sufficient resealability and sufficient force for holding the piercing device, and to avoid excessive force for the insertion of the piercing device. In addition or alternatively, in further preferred embodiments, the thickness of septum 42 measured in the direction in which it is pierced is 1.5 mm or more, preferably 2 mm or more and/or 5 mm or less, preferably 4 mm or less. Preferably, the proximal surface of the septum 42 is flat or has a rounded cavity or a pattern.

Preferably, the septum 42 is formed by means of overmoulding, in particular together with the valve member 52 - either as one overmoulded volume, i.e. by injecting the material of the elements 42 und 52 via a single injecting point, or as two separate volumes, i.e. by injecting the material of the elements 42 und 52 via at least two injection points. Such two independent volumes may optionally be connected by a sprue 6.

Alternatively, the septum 42 is not formed in situ by overmoulding but as a separate part that is inserted in the port housing 41 and fixed in place, e.g. by using a welding technique, in particular laser welding. Particularly if the septum 42 is formed as a separate part, the septum 42 may comprise a material including pigments and/or dyes capable of absorbing electromagnetic radiation in order to improve the usability of laser welding for connecting the septum to the port housing 41.

In specific embodiments, the elastic seal member 42 comprises at least one thermoplastic elastomer, more preferably at least one thermoplastic elastomer selected from the group consisting of styrenic block copolymers, thermoplastic polyolefin elastomers, thermoplastic vulcanisates, thermoplastic polyurethanes, thermoplastic copolyester, and thermoplastic polyamides, wherein the septum 42 is most preferably formed in situ by overmoulding.

In addition or alternatively, in further preferred embodiments, the elastic seal member 42 comprises an elastic material having a hardness between 25 and 55 shore A.

In the embodiment shown in Figures 13 and 14, the outer surface 421 (distal surface) of the elastic seal member 42 is essentially flat having only minor protrusions and/or recesses. Optionally, it may have a small recess, e.g. in the centre portion of the surface 421 indicating the location where the user should pierce the elastic seal member.

In the embodiment shown in Figures 13 and 14, the opening of the port housing 41 is defined by the front surface of the port housing 41. That is, the front surface is the edge surface 412 of the port housing 41. In the embodiment shown in Figures 13 and 14, the edge surface 412 has a circular ring-like shape. In alternative embodiments, the edge surface may have a different shape, e.g. an elliptical ring-like shape. In the embodiment shown in Figures 13 and 14, the edge surface is flat and perpendicular to the distal-proximal direction, i.e. the direction in which the piercing device is to be pierced through the septum 42. In alternative embodiments, the edge surface 412 may have a different shape and/or a different orientation. For instance, it may be rounded and/or tapered (i.e. inclined with respect to the distal-proximal direction).

In the embodiment shown in Figures 13 and 14, the outer surface 421 (distal surface) of the septum 42 is flush with the edge surface 412.

That is, the edge surface 412 and the distal surface 421 of the septum 42 merge continuously into each other so that, along the circumference of the septum 42, no step is formed in the area where the elastic seal member and the port housing meet each other.

In alternative embodiments, the septum 42 is not entirely flush with the edge surface 412, but only essentially flush. This means a step of only 1 mm or less, preferably 0.5 mm or less, more preferably 0.25 mm, most preferably 0.1 mm or less is formed between the distal surface of the septum 42 and the edge surface 412.

A peel-off foil 44 may be arranged atop of the elastic seal member 42. The peel-off foil 44 may be a foil comprising plastic and/or metallic materials such as an aluminium-plastic compound foil. The peel-off foil 44 may be connected to the cap 1 by welding onto the edge surface 412 of the port housing 41.

The term "peel-off foil" indicates that preferably, the foil may be removed by the user by hand without the aid of any tools.

The peel-off foil 44 is removed before a piercing device is pierced through the elastic seal member 41. The peel-off foil 44 seals and protects the elastic seal member 42, and in particular provides tamper evidence because the peel-off foil makes it easy to determine whether the elastic seal member 42 is damaged or has already been punctured. Furthermore, the peel-off foil 44 provides additional tightness for the first port 4 and helps to ensure its mechanical integrity, for example, when the cap 1 is subjected to mechanical stress.

The second port 5 may have an equivalent peel-off foil 54.

Preferably, the body 2 comprises a polyolefin and more preferably consists thereof. In particular a material selected from the group consisting of polyethylene, polypropylene and ethylene-propylene copolymers or a mixture of multiple materials selected therefrom are used as polyolefin for the body 2.

In the embodiments shown in Figures 13 and 14, the proximal-distal direction for the first port 4 and the proximal-distal direction for the second port 5 are not parallel to each other but enclose an acute angle. In other words, the first port 4 and the second port 5 are in an angled arrangement.

The angled arrangement of the first port 4 and the second port 5 makes it possible or at least easier that a piercing device and a fluid manipulation device are simultaneously connected to the first port 4 and second port 5, respectively.

When the container 100 being provided with the cap 1 according to the invention is used for administering a liquid 300 to a patient, the container 100 and the cap 1 are usually oriented upside-down compared to the orientation shown in Fig. 15. Hence, the proximal-distal direction for the first port 4 is vertical whereas the proximal-distal direction for the second port 5 is inclined. The inclined orientation of the latter may make it easier for the user to administer a substance through the second port 5, e.g. by using a syringe.

## Claims

1. Valve (5), comprising
- a valve housing (51) and
- an elastic valve member (52) accommodated in the valve housing (51) and configured to close the valve (5) when the valve (5) is in a closed operating state,
wherein the valve member (52) comprises a valve opening (522),
wherein the valve opening (522) is configured to be closed when the valve (5) is in the closed operating state and to provide fluid passage through the valve (5) when the valve (5) is in an open operating state, and
wherein the valve member (52) is overmoulded into the valve housing (51).

2. Valve according to claim 1,
wherein the bond between the valve member (52) and the valve housing (51) is fluid-tight,
wherein preferably at least a portion of the valve member (52) is bonded to the valve housing (51) along the entire circumference of the valve housing (51).

3. Valve (5) according to any of claims 1 to 2,
wherein the valve member (52) comprises at least one thermoplastic elastomer, preferably at least one thermoplastic elastomer selected from the group consisting of styrenic block copolymers, thermoplastic polyolefin elastomers, thermoplastic vulcanizates, thermoplastic polyurethanes, thermoplastic copolyester, and thermoplastic polyamides,
wherein preferably the valve member (52) material has a hardness between 25 and 55 shore A, preferably between 35 and 45 Shore A.

4. Valve (5) according to any of claims 1 to 3,
wherein the valve housing (51) is made of a material having a tensile modulus and/or flexural modulus of at least 700 MPa, and/or
wherein said material of the valve housing (51) comprises a polyolefin, preferably polypropylene and/or polyethylene, and/or
wherein said material of the valve housing (51) comprises an engineering plastic, in particular polyamide, polycarbonate and/or polystyrene.

5. Valve (5) according to any of claims 1 to 4,
wherein the valve member (52) is configured to provide a fluid-tight connection with a liquid manipulation device, wherein preferably the valve opening (522) is a self-sealing valve opening (522), wherein more preferably a self-sealing channel (522) for a male connector, in particular a self-sealing slit (522), is formed in the valve member (52), and/or
wherein a lubricant is provided in the valve opening (522).

6. Valve (5) according to any of claims 1 to 5,
wherein the valve member (52) is formed in a way to tightly connect to a male connector, said male connector preferably being designed according to any sub-part of the ISO 80369 series of standards, in particular a male Luer connector according to ISO 80369-7 or a male connector according to ISO 80369-6, and/or
wherein optionally the valve housing (51) comprises a thread structure (53), preferably a thread structure (53) according one of the sub-parts of the ISO 80369 series of standard.

7. Valve (5) according to any of claims 1 to 6,
wherein an outer surface (521) of the valve member (52) is essentially flush or entirely flush with an edge surface (512) of the valve housing (51), or
wherein the outer surface (521) of the valve member (52) projects beyond the edge surface (512) of the valve housing (51).

8. Method of manufacturing a valve (5), in particular a valve (5) according to any of claims 1 to 7, the method comprising the steps:
A) manufacturing or providing a valve housing (51),
B) overmoulding an elastic valve member (52) into the valve housing (51),
D) optionally arranging a peel-off foil (54) atop the valve member (52),
wherein preferably in step A, the valve housing (51) is manufactured through moulding.

9. Cap (1) for a fluid container (100), in particular for a container (100) for a medical fluid (300), comprising:
(i) a body (2) comprising a container connection portion (3) adapted to be tightly connected to a container opening (201) of a container body (200),
(ii) a first port (4), comprising a port housing (41),
wherein the first port (4) further comprises an elastic seal member (42) accommodated in the port housing (41) and sealingy closing the port housing (41),
wherein preferably the seal member (42) is formed as a pierceable septum (42), and
(iii) a second port (5) comprising a valve according to any of claims 1 to 7.

10. Cap (1) according to claim 9,
wherein the seal member (42) is overmoulded into the port housing (41),
wherein preferably the seal member (42) and the valve member (52) form a single elastic member overmoulded onto the body (2),
wherein more preferably the seal member (42) and the valve member (52) are connected through a sprue (6).

11. Cap (1) according to any of claims 9 to 10,
wherein the first port (4) is an administration port, wherein preferably the seal member (42) is adapted to be pierced by a spike of an administration device selected from the group consisting of infusion set, transfusion set, and transfer device such that a fluid connection between the interior of the fluid container (100) and the administration device is provided, wherein more preferably the infusion set is an infusion set according to ISO 8536-4, the transfusion set is a transfusion set according to ISO 1135-4, and the transfer device is a transfer device according to ISO 22413, and/or
wherein the second port (5) is a needle-free port, wherein in particular the second port (5) is a medication port adapted to provide a fluid connection between the interior of the fluid container (100) and a fluid manipulation device, preferably a syringe having a male connector, more preferably a male connector according to any sub-part of the ISO 80369 series of standards, most preferably a male Luer connector according to ISO 80369-7 or a male connector according to ISO 80369-6, and/or
wherein the valve housing (51) is formed monolithically with the body (2), and/or
wherein the port housing (41) is formed monolithically with the body (2).

12. Cap (1) according to any of claims 9 to 11, wherein the cap (1) is defined by anyone of the following structural features (i) to (viii), respectively alone or in combination:
(i) an outer surface (421) of the seal member (42) is essentially flush or entirely flush with an outer edge surface (412) of the port housing (41),
(ii) the outer surface (421) of the seal member (42) has a concave portion indicating the piercing point and preferably is essentially flat or entirely flat otherwise,
(iii) the seal member (42) comprises at least one thermoplastic elastomer, preferably at least one thermoplastic elastomer selected from the group consisting of styrenic block copolymers, thermoplastic polyolefin elastomers, thermoplastic vulcanizates, thermoplastic polyurethanes, thermoplastic copolyester, and thermoplastic polyamides,
(iv) the seal member (42) comprises an elastic material having a hardness between 25 and 55 shore A,
(v) the body (2) comprises a polymer material having a tensile modulus and/or flexural modulus of at least 700 MPa,
(vi) the first port (4) and the second port (5) are arranged such that an administration device and a fluid manipulation device are simultaneously connectable to the first port (4) and second port (5), respectively,
(vii) the first port (4) and the second port (5) are in an angled arrangement,
wherein preferably the first port (4) and the second port (5) are arranged such that an angle between a direction of insertion of the administration device into the first port (4) and a direction of insertion of the fluid manipulation device into the second port (5) is between 15 and 45 degrees, in particular between 25 and 35 degrees,
(viii) the angle between a direction of insertion of the administration device into the first port (4) and a direction vertical to an opening formed by the container connection portion (3) is between 0 degrees and 30 degrees, preferably between 0 degrees and 10 degrees.

13. Cap (1) according to any of claims 9 to 12,
wherein a peel-off foil (44) is arranged atop the seal member (42) seen in a direction of insertion of an administration device, in particular welded to the port housing (41) and/or
wherein a peel-off foil (54) is arranged atop the valve member (52) seen in a direction of insertion of a liquid manipulation device, in particular welded to the valve housing (51),
wherein preferably the peel-off foil (44, 54) arranged atop the seal member (42) and/or atop the valve member (52) seals the first port (4) and/or the second port (5), respectively and/or
wherein preferably the peel-off foil (44, 54) arranged atop the seal member (42) and/or atop the valve member (52) provides tamper evidence,
wherein preferably the peel-off foil (44, 54) arranged atop the seal member (42) and/or atop the valve member (52) provides tightness and integrity prior to use.

14. Fluid container (100), in particular fluid container (100) for a medical fluid (300), comprising
- a hollow container body (200) having a container body opening portion (201)
- a cap (1) according to any of claims 9 to 13, wherein the body (2) of the cap (1) is in fluid-tight connection with the opening portion (201),
wherein preferably the container body (200) is defined by anyone of the following structural features (i) to (vi), respectively alone or in combination:
(i) the container body (200) is a semi-rigid container body (200),
(ii) the container body (200) is collapsible,
(iii) the container body (200) comprises a polyolefin material, wherein more preferably at least a wall of the container body (200) is made of polypropylene and/or polyethylene, in particular PE-LD,
(iv) the container body (200) is at least partly filled with a fluid (300), in particular a medical fluid (300),
(v) wherein the container body (200) is manufactured using extrusion blow molding, injection stretch blow moulding, or blow-fill-seal technology,
(vi) wherein the container body (200) is a collapsible flexible bag, preferably made of multilayer film.

15. Method of manufacturing a cap (1) for a fluid container (100), in particular for a container (100) for a medical fluid (300), the cap (2) comprising a body (2), a first fluid port (4) with a port housing (41) and an elastic seal member (42) and a second fluid port (5) comprising a valve (5) with a valve housing (51) and an elastic valve member (52),
the method comprising the steps:
A) manufacturing or providing the body (2), the port housing (41), and the valve housing (51), preferably manufacturing or providing the body (2) with which at least one of the port housing (41) and the valve housing (51) is integrally formed,
B) overmoulding the seal member (42) into the port housing (41),
C) overmoulding the valve member (52) into the valve housing (51),
D) optionally arranging a peel-off foil (44, 54) atop the seal member (42) and/or atop the valve member (52),
wherein preferably steps B and C are at least in part simultaneously performed, and/or
wherein preferably the seal member (42) and the valve member (52) are formed as a single volume, optionally joined by at least one sprue (6).
